# EUROPÄISCHE PATENTSCHRIFT

(11) **EP 2 427 231 B1**
(45) Veröffentlichungstag und Bekanntmachung des Hinweises auf die Patenterteilung: **05.11.2014**
(21) Anmeldenummer: 10719917.6
(22) Anmeldetag: 06.05.2010
(51) Int. Cl.: A61M 1/16

(54) **ABDECKUNGSEINRICHTUNG ZUM ABDECKEN EINES FUNKTIONSABSCHNITTS EINER BEHANDLUNGSVORRICHTUNG, BEHANDLUNGSVORRICHTUNG SOWIE VERFAHREN**
COVER DEVICE FOR COVERING A FUNCTIONAL SEGMENT OF A PROCESSING DEVICE, PROCESSING DEVICE, AND METHOD
DISPOSITIF DE PROTECTION POUR RECOUVRIR UNE PARTIE FONCTIONNELLE D'UN APPAREIL DE TRAITEMENT, APPAREIL DE TRAITEMENT ET PROCÉDÉS

(30) Priorität: 07.05.2009 DE 102009020343
(43) Veröffentlichungstag der Anmeldung: 14.03.2012
(73) Patentinhaber: Fresenius Medical Care Deutschland GmbH, 61352 Bad Homburg (DE)
(72) Erfinder: EGGERT, Ilona, 60528 Frankfurt (DE); FIEDLER, Johannes, 97618 Hohenroth (DE); LUCKAU, Michael, 53797 Lohmar (DE); SCHORR, Rainer, 97478 Knetzgau (DE)
(74) Vertreter: Bobbert & Partner
(86) Internationale Anmeldenummer: PCT/EP2010/002787
(87) Internationale Veröffentlichungsnummer: WO 2010/127857

(56) Entgegenhaltungen:
- US-A- 5 928 177

## Beschreibung

Die vorliegende Erfindung betrifft eine Abdeckungseinrichtung zum Abdecken eines Funktionsabschnitts einer Behandlungsvorrichtung gemäß dem Oberbegriff des Anspruchs 1. Sie betrifft ferner eine Behandlungsvorrichtung gemäß dem Oberbegriff des Anspruchs 5 und ein Abdeckungselement gemäß Anspruch 13. Ferner schlägt die Erfindung ein Verfahren gemäß dem Oberbegriff des Anspruchs 15 vor.

Funktionsabschnitte von Behandlungsvorrichtungen, wie beispielsweise medizinischen Behandlungsvorrichtungen, werden regelmäßig mittels Abdeckungselementen geschützt, welche sich öffnen lassen.

Die US 5,928,177 A offenbart ein schwenkbare Abdeckung für eine Dialysiervorrichtung.

Aufgabe der vorliegenden Erfindung ist es, eine weitere Abdeckungseinrichtung mit einem Abdeckungselement zum Abdecken eines Funktionsabschnitts einer Behandlungsvorrichtung anzugeben.

Die Aufgabe der vorliegenden Erfindung wird durch eine Abdeckungseinrichtung mit den Merkmalen des Anspruchs 1 gelöst.

Die erfindungsgemäße Abdeckungseinrichtung weist wenigstens ein Abdeckungselement zum Abdecken wenigstens eines Funktionsabschnitts einer Behandlungsvorrichtung mit wenigstens einer Scharnierachse, mittels welcher das Abdeckungselement drehbar direkt oder indirekt an der Behandlungsvorrichtung befestigbar ist, auf.

Die Abdeckungseinrichtung weist zudem wenigstens eine Verbindungseinrichtung auf, mittels welcher die Scharnierachse mit dem Abdeckungselement oder mit der Behandlungsvorrichtung verrastbar ist.

Der Begriff "Abdeckungselement", wie er hierin verwendet wird, bezeichnet ein Bauteil, welches dazu dient, den Funktionsabschnitt der Behandlungsvorrichtung vollständig oder in wenigstens einem Abschnitt des Funktionsabschnitts abzudecken.

Der Begriff "Funktionsabschnitt" bezeichnet eine oder mehrere Komponenten, welche zum Ausüben bzw. Ausführen einer während einer Behandlung erforderlichen oder gewollten Funktion oder zum Aufnehmen einer externen Funktionseinrichtung vorgesehen sind. Die Begriffe "Funktionsabschnitt" und (insbesondere externe) "Funktionseinrichtung" sind daher im Folgenden als Synonyme zum Zwecke der Beschreibung der vorliegenden Erfindung zu verstehen, wo immer dies vom Fachmann als sinnvoll erkannt wird.

Eine "Behandlungsvorrichtung" im Sinne der vorliegenden Erfindung kann eine medizintechnische Anordnung, wie beispielsweise eine Blutbehandlungsvorrichtung, zum Beispiel eine Dialysiervorrichtung, eine Hämodialysiervorrichtung, eine Hämodiafiltrationsvorrichtung oder dergleichen, eine Anordnung in der Labortechnik, eine Anordnung in der Arznei- oder Lebensmittelherstellung oder dergleichen sein.

Der Begriff "Scharnierachse" bzw. Scharnierwelle bezeichnet eine Komponente, welche - beispielsweise in Form eines Zapfens, eines Stabs, einer Stange - als Gelenk fungiert. Das Abdeckungselement kann drehbar bzw. schwenkbar an der Behandlungsvorrichtung befestigt und/oder gelagert sein und den Funktionsabschnitt abdecken. Die Begriffe "Scharnierachse" und "Scharnierwelle" sind bei der Beschreibung der vorliegenden Erfindung als Synonyme zu verstehen, wo immer der Fachmann die Begriffe als austauschbar erkennt. Die Erfindung soll nämlich nicht dadurch beschränkt sein, dass eine Scharnierachse als eine nicht rotierende Einrichtung, die Scharnierwelle als rotierende Einrichtung verstanden werden könnte.

Der Begriff "Verbindungseinrichtung", wie er hierin verwendet wird, bezeichnet ein Element bzw. einen Abschnitt, welcher zu seiner Verbindung mit der Scharnierachse und/oder zum Aufnehmen derselben oder wenigstens eines Abschnitts hiervon geeignet ist. Die Verbindungseinrichtung kann hierzu ausgestaltet und vorgesehen sein.

Die Scharnierachse kann in die Verbindungseinrichtung(en) eingerastet oder eingeschnappt werden.

Durch Verrasten oder Verschnappen der Scharnierachse mit der Verbindungseinrichtung kann das Abdeckungselement beweglich mit der Behandlungsvorrichtung verbunden werden.

Die Begriffe "Verrasten" oder "Verschnappen" bzw. "Einrasten" oder "Einschnappen", wie sie hierin verwendet werden und synonym verwendet werden, bezeichnen ein Einklinken oder Einklicken oder Einhängen oder Verhaken der Scharnierachse in die bzw. mit der Verbindungseinrichtung (und umgekehrt). Dies kann direkt oder indirekt erfolgen. Von der Erfindung mit umfasst ist daher eine Ausführungsform, in welcher am Vorgang des Verrastens oder Verschnappens wenigstens ein weiteres Bauteil neben der Scharnierachse und der Verbindungseinrichtung derart beteiligt ist, dass die Scharnierachse und die Verbindungseinrichtung im verrasteten oder verschnappten Zustand keinen direkten Kontakt miteinander haben. Das weitere Bauteil kann beispielsweise als Muffe oder Lagerelement ausgestaltet sein.

Vorteilhafte Weiterentwicklungen der vorliegenden Erfindung sind jeweils Gegenstand der Unteransprüche.

Die erfindungsgemäße Abdeckungseinrichtung kann derart ausgestaltet sein, dass das Verrasten oder Verschnappen vorzugsweise ohne Einsatz von Werkzeugen (von den Händen des Bedienenden abgesehen) durchführbar ist, worin ein Vorteil der vorliegenden Erfindung besteht.

Das Verrasten oder Verschnappen kann vorzugsweise durch ein Heranführen der Verbindungseinrichtung an die Scharnierachse (oder umgekehrt) allein in einer Richtung senkrecht zu einer Ebene, in welcher die Scharnierachse liegt oder zu welcher diese parallel ist, erfolgen. Dies erlaubt ein Verrasten oder Verschnappen in gerader Linie, also bei geringem Platzangebot für den Verrastvorgang und schlechter Zugänglichkeit aufgrund baulicher Rahmenbedingungen.

Das Abdeckungselement kann beispielsweise als Deckel, Klappe oder Tür ausgestaltet sein. Es kann beispielsweise mit einem Gussverfahren, beispielsweise einem Spritzgussverfahren, hergestellt sein.

Das Abdeckungselement kann vollständig oder abschnittsweise aus PET (Polyethylenterephthalat), z.B. dem Eastar Copolymer DN011 der Firma Eastman (Eastman Chemical Company, Kingsport, TN, USA) und/oder PSU (Polysulfon), gefertigt sein.

Das Abdeckungselement kann in einer bevorzugten Ausführungsform des erfindungsgemäßen Abdeckungselements Einfassungen und/oder Eingriffe und/oder Abhebungen bzw. Vorsprünge bzw. Hakenelemente, beispielsweise zum komfortablen und/oder leichten Öffnen bzw. Aufklappen des Abdeckungselements zum Zugänglichmachen des Funktionsabschnitts, aufweisen.

Die Scharnierachse kann kraft- und/oder form- und/oder stoffschlüssig mit dem Abdeckungselement integriert sein.

Die Scharnierachse kann beispielsweise in einem Formteil mit dem Abdeckungselement während der Herstellung desselben gebildet werden.

Die Verbindungseinrichtung kann die Funktion eines Scharnierlagers mit einem Freiheitsgrad (Drehbewegung des Abdeckungselements um die Scharnierachse bzw. -welle - oder umgekehrt) ausüben.

Die Verbindungseinrichtung kann beispielsweise ein Scharnierlager mit der Artikelbezeichnung Hostaform C9021 der Firma Celanese/Ticona sein.

Die mit der Verbindungseinrichtung verrastete oder verschnappte Scharnierachse oder -welle kann ferner mittels eines Stifts, einer Verzurrung, einer Lasche, einer Spanneinrichtung, einer Verkapselung und dergleichen zusätzlich an der Verbindungseinrichtung gesichert und in ihrer Position fixiert werden.

Die Verbindungseinrichtung kann an einem Stator oder einem anderen Abschnitt der Behandlungsvorrichtung vorgesehen sein. Sie kann kraft- und/oder form- und/oder stoffschlüssig mit der Behandlungsvorrichtung oder dem Stator oder dem anderen Abschnitt verbunden sein.

Unter dem Begriff "Stator" ist vorzugsweise ein feststehender maschinenseitiger Teil, d.h. ein Abschnitt an der Behandlungsvorrichtung zu verstehen.

Der Funktionsabschnitt kann optional Bestandteil des Stators sein, muss aber nicht. Die Verbindungseinrichtung kann direkt am Stator oder an dem feststehenden Funktionsabschnitt befestigt sein. Sie kann ein feststehendes Bauelement sein.

Mit einer Befestigung der Verbindungseinrichtung am Stator kann gleichermaßen die Befestigung an einem feststehenden Funktionsabschnitt gemeint sein.

Das oder die Scharniere können direkt am Stator oder am Befestigungsabschnitt befestigt sein. Sie können in jedem Fall aber feststehend angeordnet ein.

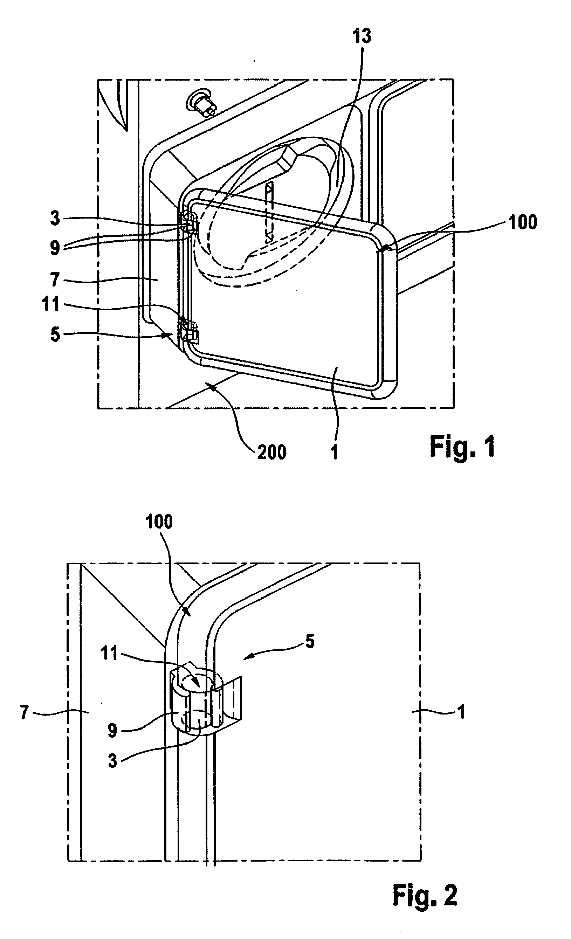

Ein Beispiel für eine derartige Anordnung ist in Fig. 1 und abschnittsweise vergrößert in Fig. 2 der angehängten Zeichnung dargestellt.

In der Ausführungsform der vorliegenden Erfindung ist die Verbindungseinrichtung auf der Seite des Abdeckungselements vorgesehen und die Scharnierachse ist auf der Seite der Behandlungsvorrichtung vorgesehen.

Die Verbindungseinrichtung kann kraft- und/oder form- und/oder stoffschlüssig mit dem Abdeckungselement integriert sein. Sie kann beispielsweise in einem Formteil mit dem Abdeckungselement während der Herstellung desselben gebildet werden.

Die Scharnierachse kann an einem Stator oder einem anderen Abschnitt der Behandlungsvorrichtung vorgesehen sein. Sie kann kraft- und/oder form- und/oder stoffschlüssig mit dem Stator oder dem anderen Abschnitt verbunden sein.

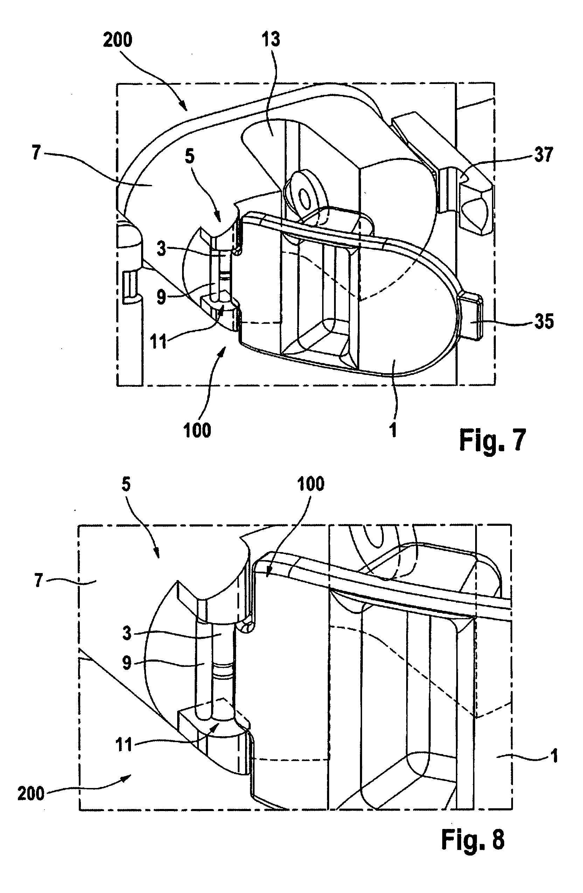

Ein Beispiel für eine derartige Anordnung ist in Fig. 7 und abschnittsweise vergrößert in Fig. 8 der angehängten Zeichnung dargestellt.

In einer weiteren Ausführungsform ist das Abdeckungselement insbesondere zerstörungsfrei lösbar mit der Behandlungsvorrichtung verbindbar oder verbunden.

Das Lösen des Abdeckungselements kann durch Entrasten oder Entschnappen der Scharnierachse aus der Verbindungseinrichtung oder umgekehrt erreicht werden.

Das Abdeckungselement kann beispielsweise vom Bedienpersonal der Behandlungsvorrichtung abgezogen, d.h. mittels Aufbringen einer mechanischen Kraft ausgeschnappt bzw. entschnappt/entrastet werden und/oder durch Herausdrücken der Scharnierachse aus der Verbindungseinrichtung von der Behandlungsvorrichtung gelöst werden.

In der erfindungsgemäßen Ausführungsform der Abdeckungseinrichtung weist die Verbindungseinrichtung wenigstens eine Buchse bzw. Buchseneinrichtung zum Aufnehmen wenigstens eines Abschnitts der Scharnierachse oder zu deren Lagerung auf.

Der Begriff "Buchseneinrichtung", wie er hierin verwendet wird, bezeichnet ein Rohrmantelelement mit einem beliebig geöffneten, jedoch vorzugsweise mehr als den halben Umfang der Scharnierachse umschließenden Mantelelement oder Maul.

Die Buchseneinrichtung formt somit eine Öffnung in einen gebildeten teiloffenen Innenraum aus.

In die Öffnung oder in das Maul (d.h. in den Durchgang) der Verbindungseinrichtung kann die einzurastende bzw. einzuschnappende Scharnierachse passgenau bzw. mit vorzugsweise so wenig Toleranz wie geeignet bzw. technisch realisierbar und möglichst kleinem Spiel aufgenommen werden.

Der Begriff "Öffnung", wie er hierin verwendet wird, bezeichnet einen Bereich oder einen Raum, ein Volumen oder eine Aufnahme und/oder Lagerung, das/die zum Aufnehmen der Scharnierachse ausgelegt bzw. vorgesehen oder geeignet ist und durch die teilweise geschlossene innere Umfangsfläche oder Mantelfläche der Buchseneinrichtung definiert ist.

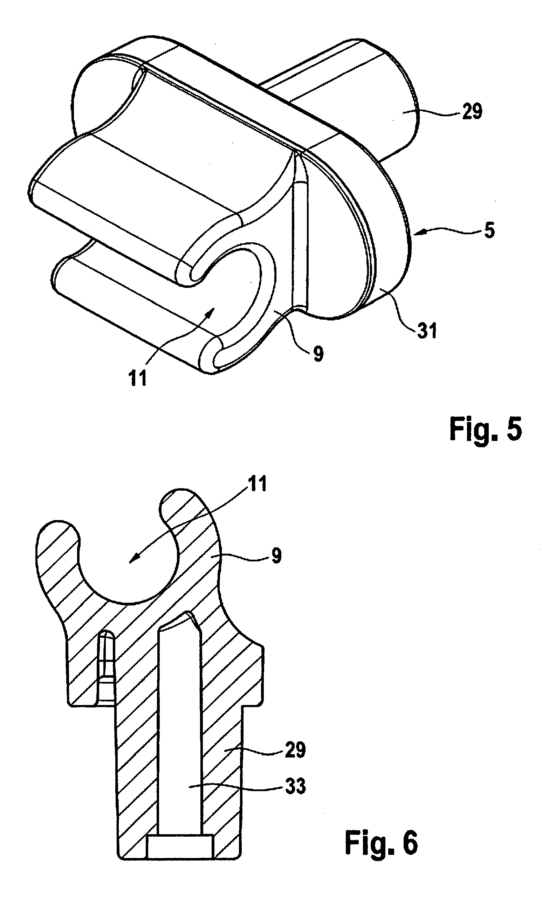

Der Innendurchmesser der Buchseneinrichtung bzw. der maximale Durchmesser der Öffnung kann dem Außendurchmesser der Scharnierachse entsprechen. Der Außendurchmesser der Scharnierachse kann jedoch auch einer Länge eines Kreissegments der Buchseneinrichtung, wie es in Fig. 6 der nachfolgenden Zeichnung veranschaulicht ist, entsprechen.

In einer weiteren Ausführungsform ist wenigstens das Abdeckungselement in wenigstens einem Abschnitt hiervon transparent.

Ein transparenter Abschnitt" kann, ohne darauf beschränkt zu sein, aus Glas, durchsichtigem Kunststoff, durchsichtiger Folie oder dergleichen ausgebildet sein oder dergleichen umfassen.

Ein transparenter Abschnitt kann opak sein.

Ein transparenter Abschnitt bzw. Bereich des Abdeckungselements kann das Betrachten oder Beobachten des Funktionsabschnitts, beispielsweise das Ablesen von Messwerten, das Überwachen eines Zustands, das Fortschreiten eines Verfahrensablaufs und dergleichen, vorteilhaft zulassen.

Der Grad der Transparenz einer derartigen Sichtfläche oder eines Sichtfensters kann durch Auswahl eines geeigneten Materials gesteuert werden. Beispielsweise kann der Gesamttransmissionsgrad des transparenten Abschnitts bei Verwenden eines Eastar Copolyester DN011-Materials der Firma Eastman 92 % betragen.

Die erfindungsgemäße Aufgabe wird ferner durch eine Behandlungsvorrichtung gemäß Anspruch 5 gelöst. Die mit der erfindungsgemäßen Abdeckungseinrichtung erzielbaren Vorteile sind ungeschmälert auch mit der erfindungsgemäßen Behandlungsvorrichtung erzielbar.

Die erfindungsgemäße Behandlungsvorrichtung weist wenigstens eine erfindungsgemäße Abdeckungseinrichtung, wie sie vorstehend beschrieben wurde, auf.

Unter Verwenden der erfindungsgemäßen Abdeckungseinrichtung kann das Abdeckungselement direkt mit der Behandlungsvorrichtung verbunden oder - wie in einer weiteren Ausführungsform der erfindungsgemäßen Behandlungsvorrichtung vorgesehen - über einen Stator der Behandlungsvorrichtung, welcher dazu ausgelegt und vorgesehen ist, wenigstens eine Verbindungseinrichtung oder wenigstens einen Teil einer Scharnierachse der Abdeckungseinrichtung aufzunehmen, mit der Behandlungsvorrichtung verbunden werden bzw. verbunden sein.

Die erfindungsgemäße Behandlungsvorrichtung kann eine Blutbehandlungsvorrichtung, wie beispielsweise eine Dialysiervorrichtung, zum Beispiel ausgestaltet als Hämodialysiervorrichtung, Hämofiltrationsvorrichtung, Hämodiafiltrationsvorrichtung und dergleichen, sein.

Der Funktionsabschnitt kann beispielsweise eine Aufnahme für ein Sensorelement bzw. Sensormodul, ein Schlauchsegment, ein Rohrsegment, insbesondere einen Rohrauslass und/oder - einlass, für einen Aktor, eine Schaltfläche bzw. ein Schaltelement, eine Bedienfläche bzw. ein Bedienelement, ein Steuerelement bzw. eine Steuereinrichtung, für ein Regelelement bzw. eine Regeleinrichtung, eine Gefäßöffnung, ein Kabel, eine Leitung, eine Steckdose, einen Stutzen, wie beispielsweise einen Einfüllstutzen, einen Mess- und/oder Probenentnahmeabschnitt, einen Ent- oder Belüftungsabschnitt und dergleichen sein. Der Funktionsabschnitt kann allerdings auch als eines der vorgenannten Elemente - oder ein hierzu ähnliches Element - ausgestaltet sein. Kombinationen der vorgenannten Elemente sind ebenfalls von der Erfindung mit umfasst.

Wenn die Behandlungsvorrichtung als Blutbehandlungsvorrichtung ausgestaltet ist, kann der Funktionsabschnitt, welcher mit einer erfindungsgemäßen Abdeckungseinrichtung abgedeckt werden kann, Teil eines extrakorporalen Blutkreislaufs, wie beispielsweise eine Blutkassette, ein Schlauchsegment, zum Beispiel ein Schlauchsegment einer Blutpumpe, eine Substituatpumpe und dergleichen sein.

In einer weiteren Ausführungsform kann der Funktionsabschnitt auch als Sensor, beispielsweise als Luftdetektor einer Tropfkammer, ausgestaltet sein.

Die erfindungsgemäße Aufgabe wird gleichermaßen durch ein Abdeckungselement für eine erfindungsgemäße Abdeckungseinrichtung, wie sie vorstehend beschrieben wurde, gelöst.

Das erfindungsgemäße Abdeckungselement kann vorbereitet bzw. geeignet bzw. vorgesehen sein, Teil der Abdeckungseinrichtung zu sein. Das Abdeckungselement kann mit einer Behandlungsvorrichtung oder einem Stator der Behandlungsvorrichtung oder einem am Stator vorgesehenen Funktionsabschnitt verbunden werden. Ein solcher Funktionsabschnitt kann beispielsweise ein Tropfkammerhalter sein, der zum Einlegen einer Tropfkammer als Bestandteil eines extrakorporalen Blutkreislaufs geeignet ist.

In einer Ausführungsform weist das Abdeckungselement wenigstens eine Scharnierachse auf. Die Behandlungsvorrichtung oder ein Stator der Behandlungsvorrichtung kann eine Verbindungseinrichtung aufweisen, mit welcher die Scharnierachse des Abdeckungselements verrastbar bzw. verschnappbar ist.

In einer weiteren Ausführungsform kann das Abdeckungselement wenigstens eine Verbindungseinrichtung zum Verrasten derselben mit einer Scharnierachse aufweisen. Die Scharnierachse kann Teil der Behandlungsvorrichtung oder eines Stators derselben oder eines am Stator vorgesehenen Funktionsabschnitts sein. Ein solcher Funktionsabschnitt kann beispielsweise ein Tropfkammerhalter sein, der zum Einlegen einer Tropfkammer als Bestandteil eines extrakorporalen Blutkreislaufs geeignet ist.

Das erfindungsgemäße Abdeckungselement eignet sich vorteilhaft als Ersatzteil, als Austauschteil, zum nachträglichen Ausstatten einer zunächst nicht erfindungsgemäßen Abdeckungseinrichtung oder Behandlungsvorrichtung. Daher sind die mit dem erfindungsgemäßen Abdeckungselement erzielbaren Vorteile jene, welche mit der erfindungsgemäßen Abdeckungseinrichtung erzielbar sind.

Ein Verfahren sieht das Entfernen eines Abdeckungselements eines mit einer erfindungsgemäßen Abdeckungseinrichtung versehenen Funktionsabschnitts einer Behandlungsvorrichtung vor.

Dazu kann das Abdeckungselement der Abdeckungseinrichtung durch Entrasten der Scharnierachse und der Verbindungseinrichtung voneinander entfernt werden.

Durch Entfernen des Abdeckungselements kann Bedienpersonal Zugang zum bzw. Zugriff auf den Funktionsabschnitt und damit die Möglichkeit des Bedienens, Steuerns oder Regelns der Behandlungsvorrichtung gewährt werden. Gleichzeitig oder zusätzlich kann das Abdeckungselement ausgetauscht bzw. ersetzt werden.

Die erfindungsgemäße Aufgabe wird gleichermaßen durch ein Verfahren gemäß Anspruch 15 gelöst, welches das Austauschen bzw. Ersetzen eines Abdeckungselements einer Behandlungsvorrichtung mittels Befestigen eines Ersatz-Abdeckungselements durch Verrasten wenigstens einer Scharnierachse mit wenigstens einer Verbindungseinrichtung zum drehbaren Befestigen des Abdeckungselements an der Behandlungsvorrichtung umfasst.

Die Behandlungsvorrichtung kann dazu vorzugsweise wenigstens eine erfindungsgemäße Abdeckungseinrichtung aufweisen. Sie kann allerdings auch erst durch das Verrasten des Ersatz-Abdeckungselements an ihr zu einer erfindungsgemäßen Behandlungsvorrichtung werden.

Das Ersatz-Abdeckungselement kann dem vorstehend beschriebenen Abdeckungselement entsprechen.

Die mit der erfindungsgemäßen Abdeckungseinrichtung erzielbaren Vorteile sind ungeschmälert auch mit den erfindungsgemäßen Verfahren erzielbar.

Die Abdeckungseinrichtung der vorliegenden Erfindung stellt vorteilhaft eine einfache und technisch wenig aufwendige Möglichkeit zur Verbindung eines Abdeckungselements mit einer Behandlungsvorrichtung bereit.

Unter Einsatz der erfindungsgemäßen Abdeckungseinrichtung kann eine solide Ausführung des Abdeckungselements und einer Scharnierachse oder -welle bereitgestellt werden, welche im Bedarfsfall - anders als bisherige Abdeckungseinrichtungen - in vorteilhafter Weise schnell, dennoch einfach und unkompliziert ausgetauscht oder ersetzt werden kann. Das Abdeckungselement kann durch einfache Handgriffe seitens des medizinischen Bedienpersonals selber gelöst bzw. ausgehebelt bzw. demontiert und erneut verrastet werden. Auf diese Weise kann in vorteilhafter Weise eine Stillstandzeit der Behandlungsvorrichtung, beispielsweise durch das Warten auf einen Kundendienst bedingt, vermieden werden.

Dies kann insbesondere von Vorteil sein, wenn durch das Fehlen durch Abfallen oder das Beschädigen des Abdeckungselements, wie beispielsweise einer Tür einer Tropfkammer für eine Blutbehandlungsvorrichtung, eine Gefährdung für einen Patienten bestünde oder die Behandlungssitzung mit der Behandlungsvorrichtung aufgrund des Fehlens nicht aufgenommen werden könnte oder unterbrochen oder beendet werden müsste. Dies kann zum Beispiel dann der Fall sein, wenn eine korrekte Ankopplung der Tropfkammer aufgrund des fehlenden Abdeckungselements nicht gewährleistet wäre und damit keine sichere Lufterkennung im Schlauchsystem erfolgen könnte.

Das erfindungsgemäße Abdeckungselement kann in vorteilhafter Weise eine verbesserte Reinigbarkeit des Abdeckungselements selbst als auch des durch das Abdeckungselement abdeckbaren maschinenseitigen Bereichs ermöglichen.

Da Verbindungseinrichtung und Scharnierachse der Abdeckungseinrichtung als lösbare Schnappverbindung oder Verrastverbindung ausgeführt sein können, kann das Abdeckungselement bei Überlastung der Scharnierachse durch beispielsweise Überdehnung bzw. Abknicken des Abdeckungselements durch das Bedienpersonal, einen Überdruck im Funktionsabschnitt oder dergleichen in vorteilhafter Weise einfach entschnappen bzw. entrasten oder abfallen bzw. abgesprengt werden.

In ähnlicher Weise kann das Abdeckungselement vorteilhaft leicht und mit geringem Aufwand wieder vom Mediziner montiert werden. Die in der erfindungsgemäßen Abdeckungseinrichtung vorgesehene Verbindung zwischen der Scharnierachse und der Verbindungseinrichtung kann daher in vorteilhafter Weise eine Art Sollbruchstelle - insbesondere eine, welche im Bedarfsfall noch nicht einmal Schaden leidet und daher nicht "bricht" wie bei Sollbruchstellen üblich sondern wieder verwendbar ist - darstellen.

Das Abdeckungselement kann in vorteilhafter Weise aus einem soliden und bruchfesten Material, wie beispielsweise Eastar Polyester CN011, hergestellt bzw. gefertigt sein, so dass es durch Herunterfallen nicht beeinträchtigt oder beschädigt wird.

Da entweder die Scharnierachse oder die Verbindungseinrichtung ebenfalls lösbar, wie beispielsweise mittels einer Schraube, und damit austauschbar mit der Behandlungsvorrichtung oder dem Stator der Behandlungsvorrichtung verbunden sein können, können - beispielsweise im Falle einer Beschädigung der Abdeckungseinrichtung, wie beispielsweise einem Bruch - kostenintensive Schäden am Stator oder an der Behandlungsvorrichtung selbst in vorteilhafter Weise vermieden werden. Ein oftmals teurer und/oder zeit- und/oder technisch aufwendiger Tausch des Stators oder eines anderen relevanten Abschnitts der Behandlungsvorrichtung kann somit in vorteilhafter Weise vermieden werden.

Die erfindungsgemäße Abdeckungseinrichtung kann einem Dauerbetrieb der Behandlungsvorrichtung, insbesondere einer medizinischen Behandlungsvorrichtung, wie es beispielsweise in einer Dialyseklinik gängig ist, in vorteilhafter Weise standhalten, da die erfindungsgemäß vorgesehene Verrast- bzw. Verschnappverbindung zwischen der Verbindungseinrichtung und der Scharnierachse durch wiederholtes Öffnen und Schließen des Abdeckungselements beliebig oft belastbar ist, ohne wesentlichen Verschleißerscheinungen unterworfen zu sein.

Das Abdeckungselement der vorliegenden Erfindung kann in vorteilhafter Weise leicht und einfach vollständig entfernt werden, so dass der vormals vom Abdeckungselement abgedeckte Funktionsabschnitt leicht zugänglich und bedienbar bzw. betätigbar und/oder reinigbar sein kann. Da der Funktionsabschnitt und das Abdeckungselement in vorteilhafter Weise separat voneinander reinigbar sind, kann in vorteilhafter Weise ein Beeinträchtigen oder Beschädigen des Abdeckungselements, wie beispielsweise eine vorzeitige Alterung des Abdeckungselements, durch die zum Reinigen des Funktionsabschnitts eingesetzten Chemikalien, wie Desinfektionsmittel, Säuren oder Laugen, vermieden werden.

Stillstandzeiten der Blutbehandlungsvorrichtung können somit vorteilhaft weiter verringert werden.

Mit der vorliegenden Erfindung können vorteilhaft das Erfordernis, einen Kundendienst oder Serviceeinsatz zum Austausch oder Ersatz des Abdeckungselements zu benötigen, und die damit verbundenen - oftmals langen Wartezeiten und hohen Kosten- entfallen.

Da das Abdeckungselement durch Entrasten bzw. Entschnappen der Scharnierachse und der Verbindungseinrichtung voneinander vollständig entfernbar ist, kann zudem - gegenüber im Stand der Technik gebräuchlichen Abdeckungseinrichtungen mit einem Spannmechanismus, durch welchen das Abdeckungselement in einem abdeckenden Zustand gehalten werden soll - vorteilhaft auf Hilfsmittel zum Aufhalten des Abdeckungselements während des Bedienens/Betätigens und/oder Reinigens des Funktionsabschnitts verzichtet werden.

Im Folgenden werden bevorzugte Ausführungsformen der vorliegenden Erfindung anhand der Zeichnung beschrieben. In den Figuren der Zeichnung bezeichnen identische Bezugszeichen gleiche oder identische Elemente. Es gilt:
- Fig. 1: zeigt eine perspektivische Ansicht einer Abdeckungseinrichtung
- Fig. 2: zeigt eine vergrößerte Ansicht eines Abschnitts der Abdeckungseinrichtung aus Fig. 1;
- Fig. 3: zeigt eine perspektivische Ansicht eines Abdeckungselements als Teil einer Abdeckungseinrichtung ähnlich jener der Fig. 1 und 2;
- Fig. 4: zeigt eine Vorderansicht des Abdeckungselements aus Fig. 3;
- Fig. 5: zeigt eine perspektivische Ansicht einer Verbindungseinrichtung als Teil einer Abdeckungseinrichtung;
- Fig. 6: zeigt eine Seitenansicht der Verbindungseinrichtung aus Fig. 5 im Vollschnitt;
- Fig. 7: zeigt eine Abdeckungseinrichtung gemäß einer Ausführungsform der vorliegenden Erfindung;
- Fig. 8: zeigt eine vergrößerte Ansicht eines Abschnitts der Abdeckungseinrichtung aus Fig. 7;
- Fig. 9: zeigt eine perspektivische Ansicht eines Tropfkammerhalters; und
- Fig. 10: zeigt eine perspektivische Ansicht eines weiteren Abdeckungselements als Teil einer erfindungsgemäßen Abdeckungseinrichtung.
- Fig. 11: zeigt ein Diagramm der Änderungen des dynamischen Schubmoduls in Abhängigkeit von der Temperatur des Werkstoffs HOSTAFORM C 9021 der Firma Celanese/Ticona (Celanese Corporation Headquarters, Dallas, Texas, USA);
- Fig. 12: zeigt ein Diagramm der Änderungen der Spannung in Abhängigkeit von der Dehnung des Werkstoffs HOSTAFORM C 9021 der Firma Celanese/Ticona (Celanese Corporation Headquarters, Dallas, Texas, USA) ;
- Fig. 13: zeigt ein Diagramm der Änderungen des Sekantenmoduls in Abhängigkeit von der Dehnung des Werkstoffs HOSTAFORM C 9021 der Firma Celanese/Ticona (Celanese Corporation Headquarters, Dallas, Texas, USA);
- Fig. 14: zeigt ein Diagramm der Änderungen der Spannung in Abhängigkeit von der Dehnung des Werkstoffs HOSTAFORM C 9021 der Firma Celanese/Ticona (Celanese Corporation Headquarters, Dallas, Texas, USA) unter isochronen Bedingungen bei 23 °C;
- Fig. 15: zeigt ein Diagramm der Änderungen des Kriechmoduls in Abhängigkeit von der Zeit des Werkstoffs HOSTAFORM C 9021 der Firma Celanese/Ticona (Celanese Corporation Headquarters, Dallas, Texas, USA) bei 23 °C; und
- Fig. 16: zeigt einen Extruder, welcher zur Vortrocknung des Werkstoffs HOSTAFORM C 9021 der Firma Celanese/Ticona (Celanese Corporation Headquarters, Dallas, Texas, USA) einsetzbar ist.

**Fig. 1** zeigt perspektivisch eine Abdeckungseinrichtung 100. Die Abdeckungseinrichtung 100 ist als Tür zum Abdecken eines in einer Behandlungsvorrichtung 200 angeordneten Blutschlauchsegments bzw. Blutschlauchmoduls ausgestaltet.

Die Abdeckungseinrichtung 100 weist ein Abdeckungselement 1, zwei Scharnierachsen 3 und zwei Verbindungseinrichtungen 5 auf.

Jede Verbindungseinrichtung 5 weist eine Buchseneinrichtung 9 auf, welche jeweils eine Öffnung 11 zum Aufnehmen einer Scharnierachse 3 bereitstellt.

Die Behandlungsvorrichtung 200 weist einen Stator 7 sowie einen Funktionsabschnitt 13 auf. Der Funktionsabschnitt 13 kann beispielsweise ein Aufnahmeabschnitt für ein Pumpenschlauchsegment einer Blutpumpe als Beispiel einer externen oder austauschbaren Funktionseinrichtung sein. Der Funktionsabschnitt 13 kann hingegen auch selbst ein Pumpenmodul, z.B. ein Blutpumpenmodul, sein, wie im Falle der Fig. 1.

Die Behandlungsvorrichtung 200 kann eine Blutbehandlungsvorrichtung, beispielsweise zum Durchführen einer Dialyse wie einer Hämofiltration, sein.

Die Abdeckungseinrichtung 100 ist in Fig. 1 in einem möglichen Öffnungszustand gezeigt.

Das Abdeckungselement 1 kann von einem geschlossenen Zustand der Abdeckungseinrichtung 100 über einen Bereich eines Winkels zu der Ebene der Berührfläche bzw. Auflagefläche oder Anpressfläche zwischen dem Abdeckungselement 1 und dem Stator 7 von im Wesentlichen oder genau 0° bis im Wesentlichen oder genau 180° - einschließlich Zwischenwerten dieses Bereichs - drehbar oder schwenkbar gelagert sein.

Das Abdeckungselement 1 kann über einen Winkel von 180° hinaus drehbar oder schwenkbar gelagert oder angeordnet sein; es kann vorzugsweise bei Überdehnung bzw. Überbeanspruchung der Schnappverbindung bzw. Verrastverbindung zerstörungsfrei aus den Verbindungseinrichtungen 5 lösbar sein. Zum Beispiel können die Scharnierachsen 3 aus den Buchseneinrichtungen 9 entrastbar bzw. ausschnappbar sein.

Der Funktionsabschnitt 13 kann während des Betriebs der Behandlungsvorrichtung 200 vom Abdeckungselement 1 der Abdeckungseinrichtung 100 abgedeckt sein, wie es ausschnittsweise in Fig. 2 gezeigt ist.

**Fig. 2** zeigt einen vergrößerten Ausschnitt der Abdeckungseinrichtung 100 aus Fig. 1, in welcher das Abdeckungselement 1 geschlossen ist und damit den Funktionsabschnitt 13 (in Fig. 2 nicht gezeigt) gegenüber einem Äußeren oder der Umgebung abdeckt. Der Funktionsabschnitt 13 kann am Stator 7 befestigt sein. Das Abdeckungselement 1 kann auf einem Gehäuserand bzw. äußeren Abschnitt des Stators 7 aufliegen und/oder mit diesem - insbesondere hermetisch, gegebenenfalls unter zusätzlichem Verwenden einer Abdichtungseinrichtung, wie beispielsweise eines Dichtmaterials - abschließen.

In Fig. 2 ist die Verbindungseinrichtung 5, welche die Buchseneinrichtung 9 sowie die Öffnung 11 zum Aufnehmen der Scharnierachse 3 aufweist, detaillierter dargestellt.

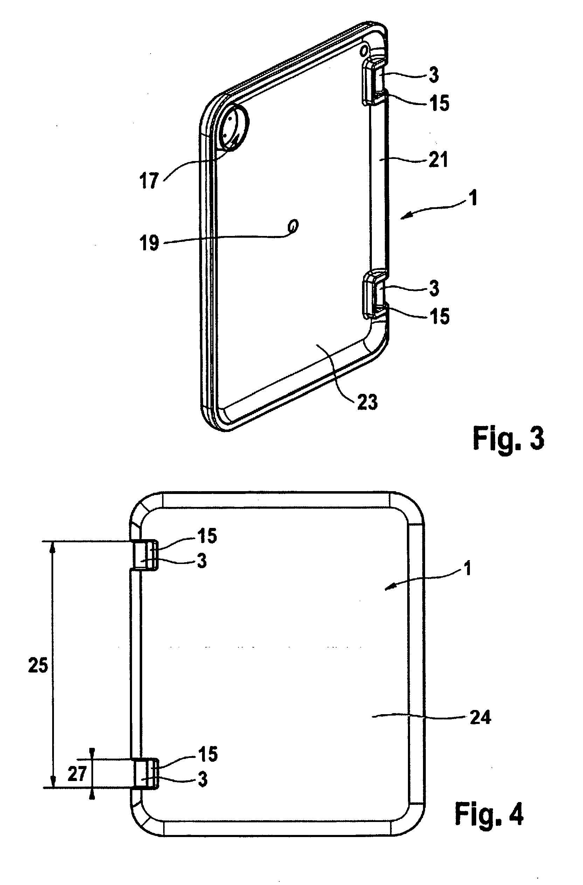

**Fig. 3** zeigt ein Abdeckungselement 1 als Teil einer ersten Abdeckungseinrichtung (in Fig. 3 nicht gezeigt).

Das Abdeckungselement 1 ist in Fig. 3 perspektivisch von der Seite der Behandlungsvorrichtung (in Fig. 3 nicht gezeigt) aus, d.h. im Wesentlichen von der Innenseite her, dargestellt.

Das Abdeckungselement 1 weist bei dieser Ausführungsform der Abdeckungseinrichtung 100 die Scharnierachsen 3 auf. Aussparungen bzw. Eingriffe 15 können dazu dienen, ein Umgreifen und damit Verschnappen bzw. Verrasten der Buchseneinrichtung(en) (in Fig. 3 nicht gezeigt) mit der Scharnierachse 3 zuzulassen.

Das Abdeckungselement 1 weist eine Einfassung 17 auf. In die Einfassung 17 kann beispielsweise ein Magnet geklebt werden, der beim Schließen der Tür einen Hallsensor im Stator der Behandlungsvorrichtung, z.B. einer Dialysevorrichtung, aktivieren kann. Die Steuerung der Behandlungsvorrichtung kann das Starten der Behandlungsvorrichtung beispielsweise erst dann zulassen, wenn das Abdeckungselement 1, beispielsweise eine Blutpumpentür, geschlossen ist. Wenn das Abdeckungselement 1 fehlt bzw. der Magnet im Abdeckungselement 1 nicht vorhanden ist, kann das Starten der Behandlungssitzung, z.B. einer Dialysebehandlung, durch die Steuerung verhindert werden.

Das Abdeckungselement 1 weist ferner einen Stangenanguss 19 auf, welcher mittig angegossen sein kann, wie beispielsweise in der Fig. 3 gezeigt ist. Der Stangenanguss 19 kann nach dem Gießen des Abdeckungselements 1 spanend bzw. spanabhebend entfernt sein. Er kann beispielsweise maximal 0,4 mm vertieft sein.

Das Abdeckungselement 1 weist einen erhöhten bzw. hoch gezogenen Rand 21 auf. Der Rand 21 kann bei geschlossener Abdeckungseinrichtung auf dem Stator (in Fig. 3 nicht gezeigt) oder jedem anderen Abschnitt der Abdeckungseinrichtung 100 oder der Behandlungsvorrichtung 200 aufliegen. Der Rand 21 bzw. das Abdeckungselement 1 können einen Zugriff auf ein Funktionselement, beispielsweise einen Rotor im Inneren der Behandlungsvorrichtung 200, verhindern. Der Rand 21 könnte den Funktionsabschnitt (in Fig. 3 nicht gezeigt) darüber hinaus hermetisch oder im Wesentlichen hermetisch und/oder flüssigkeitsdicht gegen ein Äußeres, beispielsweise gegen die Atmosphäre, abdichten oder abriegeln.

Das Abdeckungselement 1 weist eine Rückseite 23 auf, welche für einen Betrachter im geschlossenen Zustand des Abdeckungselements 1 i.d.R. nicht sichtbar ist.

Eine oder mehrere der Oberflächen des Abdeckungselements 1 können geätzt sein. Sie können gemäß "Hohenloher Formstruktur 273" geätzt sein, beispielsweise kann die Ätztiefe 0,03 mm betragen.

Eine oder mehrere der vorhandenen Flächen können jedoch auch poliert sein.

Das Abdeckungselement 1 kann frei von Auswerfermarkierungen, Fließlinien und/oder Trennverläufen sein.

Die Oberfläche des Abdeckungselements 1, beispielsweise die dem Bedienpersonal abgewandte Oberfläche der Rückseite 23, bzw. die dreidimensionale Struktur der Rückseite 23 selbst, kann frei von Lunkern, Kratzern, Riefen, Einfallstellen und dergleichen sein. Die Oberfläche kann beispielsweise maximal 0,1 mm vertiefte Auswerfermarkierungen aufweisen.

**Fig. 4** zeigt eine Vorderansicht des Abdeckungselements 1 aus Fig. 3 mit einer Front 24.

Beispielsweise kann das Abdeckungselement 1 ein Abdeckungselement sein, welches beispielsweise aus einem Material mit der Materialbezeichnung Eastar DN011 der Firma Eastman (Eastman Chemical Company, Kingsport, TN, USA) hergestellt ist. Weitere technische Angaben können Tabelle 1 der vorliegenden Anmeldung entnommen werden.

Das Abdeckungselement 1 kann in wenigstens einem Abschnitt transparent ausgeführt sein. Die Front 24 kann in wenigstens einem Abschnitt mattiert ausgeführt sein. Sie kann einen Sichtbereich aufweisen, durch welchen hindurch Bedienpersonal Einblick bzw. Draufblick auf den hinter geschlossener Abdeckungseinrichtung befindlichen Funktionsabschnitt gewährt wird.

Beispielsweise kann das Abdeckungselement 1 für eine Blutpumpentür vorzugsweise mattiert ausgeführt sein. Das Abdeckungselement 1 oder die Blutpumpentür kann jedoch auch transparent ausgeführt sein. Für die Tür eines Luftdetektors ("LD-Tür") kann das Abdeckungselement 1 beispielsweise mit einem transparenten Sichtfenster ausgestattet sein. Das Sichtfenster einer LD-Tür kann ebenso glasklar ausgestaltet sein.

Das Abdeckungselement 1 weist zwei Scharnierachsen 3 auf, deren einander abgewandte Außenseiten einen Abstand 25 von beispielsweise 101,5 mm ± 0,2 mm aufweisen können.

Die Länge 27 des Eingriffs 15 kann beispielsweise 11,5 mm +0,1 mm/-0,3 mm betragen.

**Fig. 5** zeigt eine Verbindungseinrichtung 5 als Teil einer Abdeckungseinrichtung.

Die Verbindungseinrichtung 5 weist eine Buchseneinrichtung 9 auf, welche eine Öffnung 11 zum Aufnehmen einer in Fig. 5 nicht gezeigten Scharnierachse bereitstellt.

Die Verbindungseinrichtung 5 ist über eine Führung 29 mit dem in Fig. 5 nicht gezeigten Stator einer Behandlungsvorrichtung verbindbar. Beispielsweise kann die Führung 29 in eine im oder am Stator vorgesehene Aufnahme eingeführt und die Verbindungseinrichtung 5 mittels einer Schraubverbindung mit dem Stator bzw. der Behandlungsvorrichtung verbunden werden.

Die Verbindungseinrichtung 5 kann mit einem Sitz 31 auf einer Oberfläche des Stators aufliegen.

**Fig. 6** zeigt eine Seitenansicht der Verbindungseinrichtung 5 aus Fig. 5 im Schnitt.

Die Verbindungseinrichtung 5 kann beispielsweise ein Scharnierlager oder ein Abschnitt hiervon sein, zum Beispiel ein Scharnierlager mit der Artikelbezeichnung Hostaform C9021 der Firma Celanese/Ticona.

Weitere technische Angaben können Tabelle 2 der vorliegenden Anmeldung entnommen werden.

Die Verbindungseinrichtung 5 weist die Buchseneinrichtung 9 auf, welche eine Öffnung 11 zum Aufnehmen einer Scharnierachse (in Fig. 6 nicht gezeigt) ausformt. Ein Kreissegment des Innendurchmessers der Buchseneinrichtung 9 kann beispielsweise eine Länge von 4,15 mm ± 0,05 mm aufweisen. Diese Länge kann im Wesentlichen oder genau dem Durchmesser der Scharnierachse entsprechen oder auf diesen geeignet abgestimmt sein.

Die Führung 29 weist eine Vorform 33 für ein Innengewinde für eine Schraube auf. Eine Schraube kann dazu vorgesehen sein, die Verbindungseinrichtung 5 mittels der Führung 29 an einem Stator (in Fig. 6 nicht gezeigt) bzw. einer Behandlungsvorrichtung (in Fig. 6 nicht gezeigt) zu befestigen. Hierzu kann auch eine Mehrzahl von Schrauben oder anderen Verbindungselementen vorgesehenen sein, ebenso Kombinationen unterschiedlicher Verbindungselemente. Anstelle einer Vorform 33 für ein Innengewinde für eine Schraube kann auch ein fertiges Innengewinde vorgesehen sein.

**Fig. 7** zeigt eine perspektivische Ansicht einer Abdeckungseinrichtung 100 gemäß einer Ausführungsform der vorliegenden Erfindung. Die Abdeckungseinrichtung 100 kann als Klappe zum Abdecken des Funktionsabschnitts und/oder einer Funktionseinrichtung, wie zum Beispiel einem Sensor bzw. Sensormodul, wie einem Luftdetektor einer Tropfkammer, usw. ausgestaltet sein.

Die Abdeckungseinrichtung 100 weist wiederum ein Abdeckungselement 1 auf.

Das Abdeckungselement 1 weist die Verbindungseinrichtung 5 auf, welche ihrerseits die Buchseneinrichtung 9 und die Öffnung 11 zum Aufnehmen einer Scharnierachse 3 aufweist.

Die Scharnierachse 3 ist in der Ausführungsform der Fig. 7 am Stator 7 der Behandlungsvorrichtung 200 vorgesehen.

Das Abdeckungselement 1 weist eine Verriegelungseinrichtung 35 auf, welche in eine Verriegelungsaufnahme 37 des Stators 7 zum Schließen der Abdeckungseinrichtung 100 einschnappbar oder einrastbar oder auf andere Weise mit dieser verbindbar ist.

Die Abdeckungseinrichtung 100 ist in einem geöffneten Zustand gezeigt. Das Abdeckungselement 1 kann soweit geöffnet werden, dass der Funktionsabschnitt für Bedienpersonal zugänglich, zum Beispiel bedienbar und/oder reinigbar, ist. Das Abdeckungselement kann ganz entfernt werden.

Die erfindungsgemäße Ausführungsform unterscheidet sich damit von der ersten Ausführungsform im Wesentlichen dadurch, dass - wie in Fig. 7 gezeigt - die Verbindungseinrichtung 5 am Abdeckungselement 1 und die Scharnierachse 3 am Stator 7 angeordnet ist. Die Komponenten der zweiten Ausführungsform können dabei im Wesentlichen den Komponenten der ersten Ausführungsform entsprechen. Zur Vermeidung von Wiederholungen wird daher - soweit nicht ausdrücklich angegeben - auf die vorstehend ausgeführte Beschreibung der einzelnen Komponenten in Verbindung mit der Beschreibung der ersten Ausführungsform verwiesen. Ein vorteilhafter Unterschied zwischen dem ersten Beispiel und dem Ausführungsbeispiel ist, dass die Buchseneinrichtung 9 bei geschlossenem Abdeckungselement 1 "von hinten", d.h. von der Seite der Vorrichtung aus, um die Scharnierachse 3 herumgreift.

Dies kann insbesondere vorteilhaft sein, da sich die Schnappverbindung nicht unbeabsichtigt lösen kann, wenn z.B. eine Kraft auf die Rückseite des Abdeckungselements 1 wirkt. Eine solche Kraft könnte beispielsweise von einer Funktionseinrichtung ausgehen, z.B. wenn eine externe Funktionseinrichtung (z.B. Teil eines Blutschlauchsatzes, einer Tropfkammer und dergleichen) mittels des Abdeckungselements 1 gehalten und/oder in den Funktionsabschnitt gepresst bzw. in diesem verpresst wird.

**Fig. 8** zeigt einen vergrößerten Ausschnitt der Abdeckungseinrichtung 100 der Fig. 7. Die Abdeckungseinrichtung 100 ist auch in Fig. 8 in einem geöffneten Zustand gezeigt.

In Fig. 8 ist die Verbindungseinrichtung 5, welche die Buchseneinrichtung 9 sowie die Öffnung 11 zum Aufnehmen der Scharnierachse 3 aufweist, detailliert dargestellt.

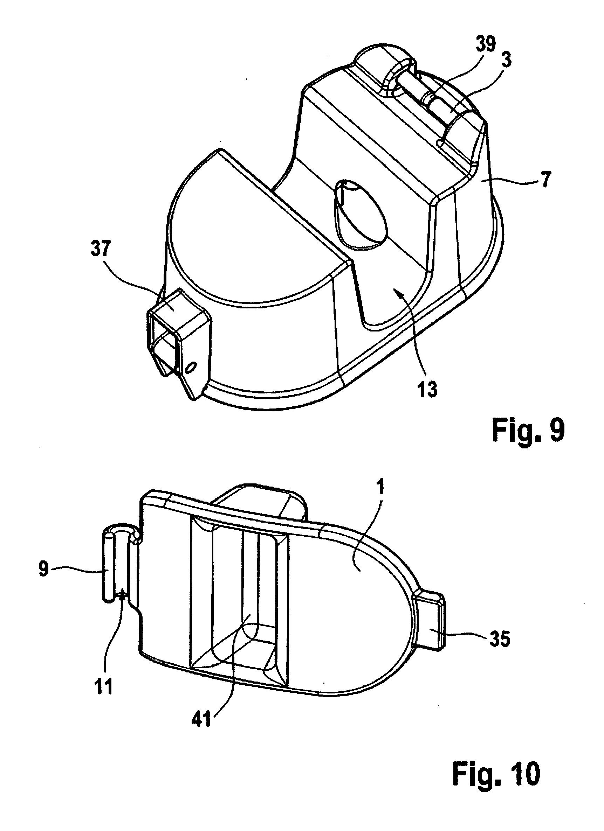

**Fig. 9** zeigt den Stator 7 in einer Ausgestaltung als Tropfkammerhalter für eine Abdeckungseinrichtung gemäß der zweiten Ausführungsform der vorliegenden Erfindung.

Der Stator 7, d.h. der Tropfkammerhalter kann an einer Behandlungsvorrichtung (in Fig. 9 nicht gezeigt) befestigt werden.

Der Stator 7 weist die Scharnierachse 3 auf. Die Scharnierachse 3 weist eine Verjüngung 39 auf. Die Verjüngung 39 in der Scharnierachse 7 kann während der Fertigung zur Positionierung der Scharnierachse 7 im Spritzgusswerkzeug dienen. Auf diese Weise kann es leicht möglich sein, die Achse zum Beispiel immer mittig zum Gehäuse einzuspritzen.

Der Stator 7 weist einen Funktionsabschnitt 13 auf.

Der Stator 7 weist eine Verriegelungsaufnahme 37 auf.

**Fig. 10** zeigt ein Abdeckungselement 1 als Teil einer Abdeckungseinrichtung gemäß der Ausführungsform der vorliegenden Erfindung.

Das Abdeckungselement 1 weist die Buchseneinrichtung 9 auf, welche in ihrem Inneren eine Öffnung 11 zum Aufnehmen einer Scharnierachse (in Fig. 10 nicht gezeigt) bereitstellt.

Das Abdeckungselement 1 weist eine Verriegelungseinrichtung 35 auf, welche mit einer Verriegelungsaufnahme (in Fig. 10 nicht gezeigt) verschnappbar bzw. verrastbar ist. Eine externe Funktionseinrichtung kann mittels der in eine Verriegelungsaufnahme eingeschnappten oder eingerasteten Verriegelungseinrichtung 35 form- und/oder kraftschlüssig verpresst werden.

Das Abdeckungselement 1 kann eine Klappe sein, die ähnliche oder gleiche Spezifikationen aufweisen kann, wie sie vorstehend bereits beschrieben wurden.

Das Abdeckungselement 1 kann transparent ausgeführt sein. Ein Sichtfenster 41 kann glasklar sein. Das Sichtfenster 41 kann eine hochglanzpolierte Werkzeugoberfläche, beispielsweise die dem Bedienpersonal zugewandte Oberfläche, aufweisen.

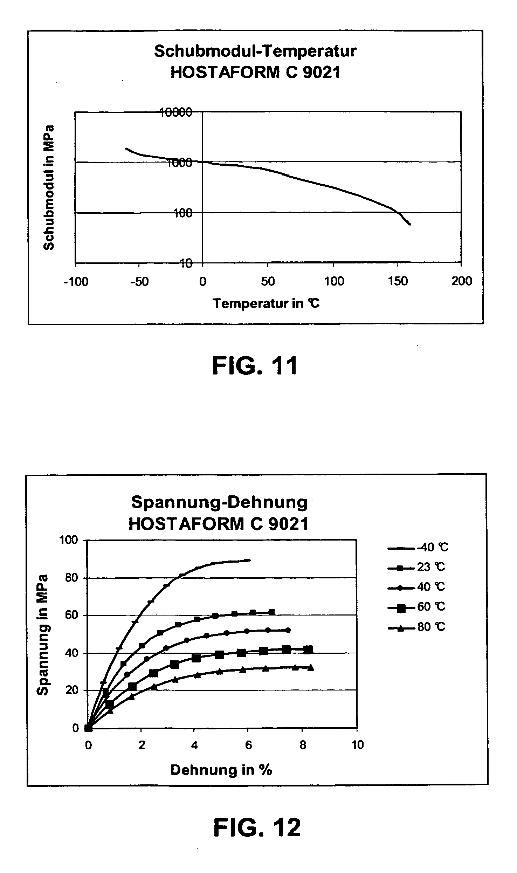

**Fig. 11** zeigt ein Diagramm der Änderungen des dynamischen Schubmoduls in Abhängigkeit von der Temperatur des Werkstoffs HOSTAFORM C 9021 der Firma Celanese/Ticona (Celanese Corporation Headquarters, Dallas, Texas, USA), welches dem Produktdatenblatt der Firma Celanese/Ticona mit Stand vom 10. Juni 2009 entnommen wurde.

**Fig. 12** zeigt ein Diagramm der Änderungen der Spannung in Abhängigkeit von der Dehnung des Werkstoffs HOSTAFORM C 9021 der Firma Celanese/Ticona (Celanese Corporation Headquarters, Dallas, Texas, USA), welches dem Produktdatenblatt der Firma Celanese/Ticona mit Stand vom 10. Juni 2009 entnommen wurde.

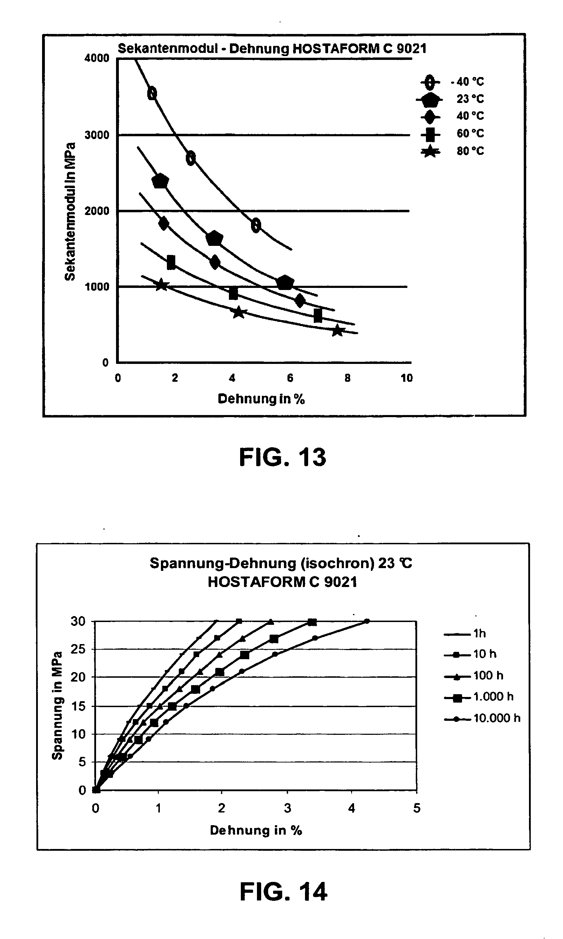

**Fig. 13** zeigt ein Diagramm der Änderungen des Sekantenmoduls in Abhängigkeit von der Dehnung des Werkstoffs HOSTAFORM C 9021 der Firma Celanese/Ticona (Celanese Corporation Headquarters, Dallas, Texas, USA), welches dem Produktdatenblatt der Firma Celanese/Ticona mit Stand vom 10. Juni 2009 entnommen wurde.

**Fig. 14** zeigt ein Diagramm der Änderungen der Spannung in Abhängigkeit von der Dehnung des Werkstoffs HOSTAFORM C 9021 der Firma Celanese/Ticona (Celanese Corporation Headquarters, Dallas, Texas, USA) unter isochronen Bedingungen bei 23 °C, welches dem Produktdatenblatt der Firma Celanese/Ticona mit Stand vom 10. Juni 2009 entnommen wurde.

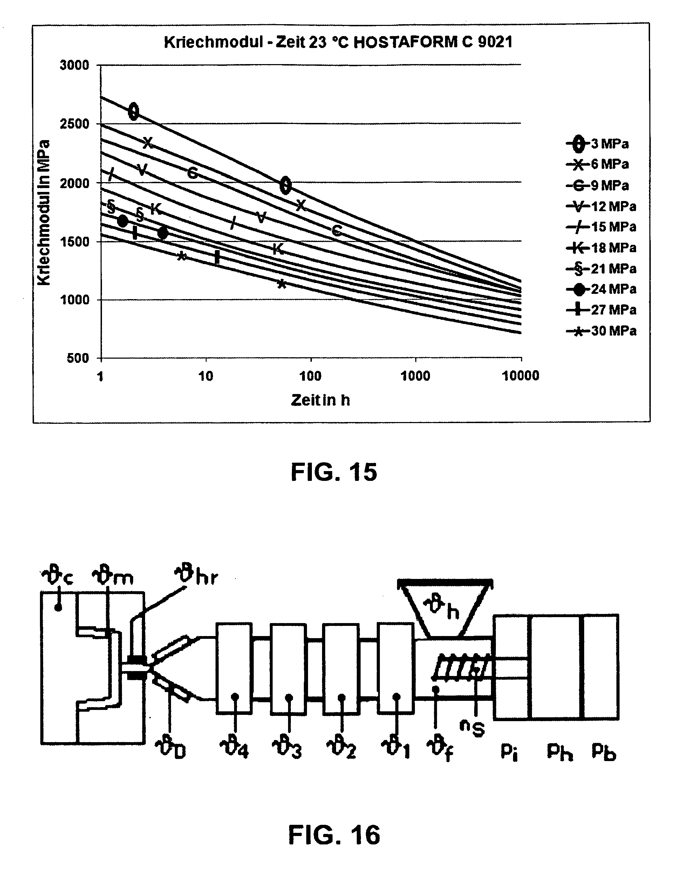

**Fig. 15** zeigt ein Diagramm der Änderungen des Kriechmoduls in Abhängigkeit von der Zeit des Werkstoffs HOSTAFORM C 9021 der Firma Celanese/Ticona (Celanese Corporation Headquarters, Dallas, Texas, USA) bei 23 °C, welches dem Produktdatenblatt der Firma Celanese/Ticona mit Stand vom 10. Juni 2009 entnommen wurde.

**Fig. 16** zeigt einen Extruder, welcher zur Vortrocknung des Werkstoffs HOSTAFORM C 9021 der Firma Celanese/Ticona (Celanese Corporation Headquarters, Dallas, Texas, USA) einsetzbar ist.

Die Vortrocknung kann folgendermaßen erfolgen:
max. zulässige Restfeuchte: 0,15 %
Trocknungszeit: 3 - 4 h
Trocknungstemperatur: 120 - 140 °C
Temperatur:

| | min (°C) | max (°C) |
|---|---|---|
| ϑ_Verteiler | 190 | 210 |
| ϑ_Wand | 80 | 120 |
| ϑ_Masse | 190 | 210 |
| ϑ_Düse | 190 | 210 |
| ϑ_Zone 4 | 190 | 210 |
| ϑ_Zone 3 | 190 | 200 |
| ϑ_Zone 2 | 180 | 190 |
| ϑ_Zone 1 | 170 | 180 |
| ϑ_Einzug | 60 | 80 |
| ϑ_Trichter | 20 | 30 |

Druck:

| | Einspritzdr. | Nachdr. | Staudruck |
|---|---|---|---|
| min (bar) | 600 | 600 | 0 |
| max (bar) | 1200 | 1200 | 40 |

Geschwindigkeit:
Einspritzgeschwindigkeit: langsam-mittel

Schneckendrehzahl:

| | | | | | |
|---|---|---|---|---|---|
| Durchmesser (mm) | 16 | 25 | 40 | 55 | 75 |
| Geschwindigkeit (rpm) | - | 150 | 100 | 70 | - |

Spritzgießen:
Auf handelsüblichen Spritzgießmaschinen mit 15-25 D langen Dreizonen-Schnecken verarbeitbar.

| | |
|---|---|
| Massetemperatur | 190-230 °C |
| Werkzeugwandtemperatur | 60-120 °C |

Folienextrusion:
Auf handelsüblichen Nutbuchsen-Extrudern mit mind. 25 D langen Kurzkompressions-Schnecken verarbeitbar.

| | |
|---|---|
| Massetemperatur | 180-190 °C |

Übrige Extrusion:
Auf handelsüblichen Nutbuchsen-Extrudern mit mind. 25 D langen Kurzkompressions-Schnecken verarbeitbar.

| | |
|---|---|
| Massetemperatur | 180-190 °C |

Profilextrusion:
Auf handelsüblichen Nutbuchsen-Extrudern mit mind. 25 D langen Kurzkompressions-Schnecken verarbeitbar.

| | |
|---|---|
| Massetemperatur | 180-190 °C |

Plattenextrusion:
Auf handelsüblichen Nutbuchsen-Extrudern mit mind. 25 D langen Kurzkompressions-Schnecken verarbeitbar.

| | |
|---|---|
| Massetemperatur | 180-190 °C |

Tabelle 1 zeigt die Kenndaten des Eastar Copolymers DN011 der Firma Eastman (Eastman Chemical Company, Kingsport, TN, USA).

Tabelle 2 zeigt die Kenndaten des Hostaform ® C 9021 POM Ungefüllt der Firma Celanese/Ticona (Celanese Corporation Headquarters, Dallas, Texas, USA).

### Tabelle 1

### Produktdatenblatt Eastar Copolyester DN011

### Produktbeschreibung

*Eastar* Copolyester DN011 ist ein glänzend durchsichtiges Polymer mit ausgezeichneter Schlagzähigkeit, chemischer Beständigkeit und geringem Schrumpfungsanteil. *Eastar* Copolyester DN011 enthält eine Formtrennung.

| **Kennwerte** | **Testverfahren^{b}** | **Typische Kennwerte, Einheiten^{c}** |
|---|---|---|
| Spezifische Dichte | D 792 | 1,23 |
| Verarbeitungsschwindung längs der Strömung | D 955 | 0,002-0,005 mm/mm (0,002-0,005 Inch/Inch) |

| **Mechanische Kennwerte** | | |
|---|---|---|
| Streckspannung | D 638 | 44 MPa (6300 Pfund pro Quadratzoll) |
| Reißfestigkeit | D 638 | 54 MPa (7800 Pfund pro Quadratzoll) |
| Streckdehnung | D 638 | 4 % |
| Reißdehnung | D 638 | 330 % |
| Zugmodul | D 638 | 1800 MPa (2,6 x 10⁵ Pfund pro Quadratzoll) |
| Biegemodul | D 790 | 1800 MPa (2,6 x 10⁵ Pfund pro Quadratzoll) |
| Biegefestigkeit | D 790 | 66 MPa (9600 Pfund pro Quadratzoll) |
| Rockwell-Härte, R-Skala | D 785 | 105 |
| Izod-Kerbschlagzähigkeit Bei 23 °C (73 °F) | D 256 | NB |
| Bei -40 °C (-40 °F) | D 256 | 77 J/m (1,4 Fluß · Pfund-Kraft/Inch) |
| Schlagzähigkeit, ungekerbt | | |
| Bei 23 °C (73 °F) | D 4812 | NB |
| Bei -40 °C (-40 °F) | D 4812 | NB |
| (Durchschlag- )Schlagfestigkeit, Energie bei max. Belastung | | 46 J (34 Fluß · Pfund-Kraft) |
| | | |
| Bei 23 °C (73 °F) | D 3763 | 46 J (34 Fluß · Pfund-Kraft ) |
| Bei -40 °C (-40 °F) | D 3763 | |

| **Thermische Kennwerte** | | |
|---|---|---|
| Formbeständigkeitstemperatur | | |
| Bei 0,455 MPa (66 psi) | D 648 | 73 °C (163 °F) |
| Bei 1,82 MPa (264 psi) | D 648 | 64 °C (147 °F) |

| **Optische Kennwerte** | | |
|---|---|---|
| Trübung | D 1003 | <1,0 % |
| Normale Lichttransmission | D 1003 | 89 % |
| Gesamttransmissionsgrad | D 1003 | 92 % |

| **Typische Verarbeitungsbedingungen** | | |
|---|---|---|
| Trocknungstemperatur | | 71 °C (160 °F) |
| Trocknungsdauer | | 6 Stunden |
| Schmelztemperatur während der Verarbeitung | | 249-271 °C (480-520 °F) |
| Werkzeugtemperatur | | 16-38 °C (60-100 °F) |

| | | |
|---|---|---|
| ^{a} Soweit nicht anders angegeben, wurden alle Tests bei 23 °C (73 °F) und 50 % relativer Feuchte durchgeführt. ^{b} Soweit nicht anders angegeben, ist das Testverfahren ASTMstandardisiert. ^{c} Die Einheiten entsprechen den gängigen SI- oder US-Einheiten. | | |

### Tabelle 2

### Produktdatenblatt HOSTAFORM ® C 9021 POM Ungefüllt

Chemische Kurzbezeichnung nach ISO 1043-1: POM Formmasse ISO 9988-POM-K,M-GNR, 03-002

### POM-Copolymer

Standard-Spritzgießtyp mit hoher Steifheit, Härte und Zähigkeit, gute chemische Beständigkeit gegen Lösemittel, Kraftstoffe und starke Alkalien sowie gute Hydrolysebeständigkeit; hoher Widerstand gegen thermischen und oxidativen Abbau.

Erfüllt EG-Richtlinie 2002/72/EU bzw. die Empfehlung XXXIII für Bedarfsgegenstände des BgVV, entspricht FDA-Regelung 21 CFR 177.2470 für Lebensmittelkontakt.

UL-Registrierung in allen Farben ab 1,5 mm Dicke als UL 94 HB, Temperatur-Index UL 746 B elektrisch 110 °C, mechanisch 90 °C, Brenngeschwindigkeit ISO 3795 und FMVSS 302 < 75 mm/min ab 1 mm Dicke.

Anwendungsbereiche: Automobilbau, Feinwerktechnik, Elektro- und Elektronikindustrie, Hausgeräte.
FDA = Food and Drug Administration (USA)
BgVV = Bundesinstitut für gesundheitlichen Verbraucherschutz und Veterinärmedizin
FMVSS = Federal Motor Vehicle Safety Standard (USA)
UL = Underwriters Laboratories (USA)

| **Physikalische Kennwerte** | **Wert** | **Einheit** | **Test Standard** |
|---|---|---|---|
| Dichte | 1410 | kg/m³ | ISO 1183 |
| Schmelzevolumenrate (MVR) | 8 | cm³/10mi n | ISO 1133 |
| MVR Prüftemperatur | 190 | °C | ISO 1133 |
| MVR Prüfbelastung | 2,16 | kg | ISO 1133 |
| Verarbeitungsschwindung - längs | 2 | % | ISO 294-4 |
| Verarbeitungsschwindung - quer | 1,8 | % | ISO 294-4 |
| Wasseraufnahme (23 °C-ges) | 0,65 | % | ISO 62 |
| Feuchtigkeitsaufnahme (23°C/50%RH) | 0,2 | % | ISO 62 |

| **Mechanische Kennwerte** | **Wert** | **Einheit** | **Test Standard** |
|---|---|---|---|
| Zug-Modul (1mm/min) | 2850 | MPa | ISO 527-2/1A |
| Streckspannung (50mm/min) | 64 | MPa | ISO 527-2/1A |
| Streckdehnung (50mm/min) | 9 | % | ISO 527-2/1A |
| Nominelle Bruchdehnung (50mm/min) | 30 | % | ISO 527-2/1A |
| Zug-Kriechmodul (1h) | 2500 | MPa | ISO 899-1 |
| Zug-Kriechmodul (1000h) | 1300 | MPa | ISO 899-1 |
| Biegemodul (23°C) | 2700 | MPa | ISO 178 |
| Charpy-Schlagzähigkeit bei 23°C | 180P | kJ/m² | ISO 179/1eU |
| Charpy-Schlagzähigkeit bei - 30°C | 160 | kJ/m² | ISO 179/1eU |
| Charpy-Kerbschlagzähigkeit bei 23°C | 6,5 | kJ/m² | ISO 179/1eA |
| Charpy-Kerbschlagzähigkeit bi -30°C | 6 | kJ/m² | ISO 179/1eA |

| **Thermische Kennwerte** | **Wert** | **Einheit** | **Test Standard** |
|---|---|---|---|
| Schmelztemperatur (10°C/min) | 166 | °C | ISO 11357-1,-2,-3 |
| DTUL bei 1,8 MPa | 104 | °C | ISO 75-1/-2 |
| Vicat Erweichungstemperatur B50 (50°C/h 50N) | 150 | °C | ISO 306 |
| Längenausdehnungskoeffizient (längs) | 1,1 | E-4/°C | ISO 11359-2 |
| Längenausdehnungskoeffizient (quer) | 1,1 | E-4/°C | ISO 11359-2 |
| Brennbarkeit bei nominal 1,6 mm | HB | class | UL 94 |
| geprüfte Probekörperdicke (1,6) | 1,5 | mm | UL 94 |
| UL Registrierung (1,6) | UL | - | UL 94 |
| Brennbarkeit bei Dicke h | HB | class | UL 94 |
| geprüfte Probekörperdicke (h) | 3 | mm | UL 94 |
| UL Registrierung (h) | UL | - | UL 94 |

| **Elektrische Kennwerte** | **Wert** | **Einheit** | **Test Standard** |
|---|---|---|---|
| Dielektrizitätszahl - 100 Hz | 4 | - | IEC 60250 |
| Dielektrizitätszahl - 1 MHz | 4 | - | IEC 60250 |
| Dielektrischer Verlustfaktor - 100 Hz | 20 | E-4 | IEC 60250 |
| Dielektrischer Verlustfaktor - 1 MHz | 50 | E-4 | IEC 60250 |
| Spezifischer Durchgangswiderstand | 1E12 | Ohm²m | IEC 60093 |
| Spezifischer Oberflächenwiderstand | 1E14 | Ohm | IEC 60093 |
| Elektrische Durchschlagfestigkeit | 35 | kV/mm | IEC 60243-1 |
| Vergleichszahl der Kriechwegbildung CTI | 600 | - | IEC 60112 |

| **Probekörperherstellbedingungen** | **Wert** | **Einheit** | **Test Standard** |
|---|---|---|---|
| Herstellbedingungen nach ISO | 9988 | - | Intern |

| **Kennwerte für rheologische Berechnungen** | **Wert** | **Einheit** | **Test Standard** |
|---|---|---|---|
| Dichte der Schmelze | 1200 | kg/m³ | Intern |
| Wärmeleitfähigkeit der Schmelze | 0,155 | W (m K) | Intern |
| Spez. Wärmekapazität der Schmelze | 2210 | J (kg K) | Intern |
| Effektive Temperaturleitf. a-effektiv | 4,85E-8 | m²/s | Intern |
| Entformungstemperatur | 165 | °C | Intern |

## Patentansprüche

1. Abdeckungseinrichtung (100) mit wenigstens einem Abdeckungselement (1) zum Abdecken wenigstens eines Funktionsabschnitts (13) einer Behandlungsvorrichtung (200) mit
- wenigstens einer Scharnierachse (3), mittels welcher das Abdeckungselement (1) drehbar an der Behandlungsvorrichtung (200) befestigbar ist, wobei
die Abdeckungseinrichtung (100) wenigstens eine Verbindungseinrichtung (5) aufweist, mittels welcher die Scharnierachse (3) mit dem Abdeckungselement (1) verrastbar ist, und wobei
die Verbindungseinrichtung (5) wenigstens eine Buchseneinrichtung (9) zum Aufnehmen wenigstens eines Abschnitts der Scharnierachse (3) oder zu deren Lagerung aufweist, wobei die Verbindungseinrichtung (5) auf der Seite des Abdeckungselements (1) vorgesehen ist und die Scharnierachse (3) auf der Seite der Behandlungsvorrichtung (200) vorgesehen ist,
**dadurch gekennzeichnet, dass**
die Buchseneinrichtung (9) bei geschlossenem Abdeckungselement (1) von der Seite der Behandlungsvorrichtung (200) aus um die scharnierachse (3) herumgreift.

2. Abdeckungseinrichtung (100) nach Anspruch 1, wobei das Abdeckungselement (1) zerstörungsfrei lösbar mit der Behandlungsvorrichtung (200) verbindbar oder verbunden ist.

3. Abdeckungseinrichtung (100) nach einem der vorangehenden Ansprüche, wobei die Scharnierachse (3) in eine Öffnung (11) einschnappbar ausgeführt ist.

4. Abdeckungseinrichtung (100) nach einem der vorangegangenen Ansprüche, wobei wenigstens das Abdeckungselement (1) in wenigstens einem Abschnitt hiervon transparent ist.

5. Behandlungsvorrichtung (200) **gekennzeichnet durch** eine Abdeckungseinrichtung (100) gemäß einem der Ansprüche 1 bis 4.

6. Behandlungsvorrichtung (200) nach Anspruch 5, welche wenigstens einen Stator (7) aufweist, welcher dazu ausgelegt und vorgesehen ist, wenigstens einen Teil einer Scharnierachse (3) der Abdeckungseinrichtung (100) aufzunehmen.

7. Behandlungsvorrichtung (200) nach Anspruch 5 oder 6, welche als Blutbehandlungsvorrichtung ausgestaltet ist.

8. Behandlungsvorrichtung (200) nach Anspruch 7, welche als Dialysiervorrichtung ausgestaltet ist.

9. Behandlungsvorrichtung (200) nach Anspruch 8, welche als Hämodialysiervorrichtung ausgestaltet ist.

10. Behandlungsvorrichtung (200) nach einem der Ansprüche 7 bis 9, wobei der Funktionsabschnitt (13) Teil eines extrakorporalen Blutkreislaufs ist.

11. Behandlungsvorrichtung (200) nach Anspruch 10, wobei der Funktionsabschnitt (13) der Behandlungsvorrichtung (100) eine Aufnahme für ein Schlauchsegment einer Blutpumpe ist.

12. Behandlungsvorrichtung (200) nach Anspruch 10, wobei der Funktionsabschnitt (13) ein Sensor ist.

13. Abdeckungselement (1) für eine Abdeckungseinrichtung (100) gemäß einem der Ansprüche 1 bis 4.

14. Abdeckungselement (1) nach Anspruch 13, welches wenigstens eine Verbindungseinrichtung (5) zum Verrasten derselben mit einer Scharnierachse (3) aufweist.

15. Verfahren zum Ersetzen eines Abdeckungselements (1) einer Behandlungsvorrichtung (200), wobei die Behandlungsvorrichtung (200) wenigstens eine Abdeckungseinrichtung (100) gemäß einem der Ansprüche 1 bis 4 aufweist, **gekennzeichnet durch** den Schritt:
- Befestigen eines Ersatz-Abdeckungselements (1) durch Verrasten wenigstens einer Scharnierachse (3) mit wenigstens einer Verbindungseinrichtung (5) zum drehbaren Befestigen des Abdeckungselements (1) an der Behandlungsvorrichtung (200).

## Claims

1. Cover means (100) having at least one cover member (1) for covering at least one functional portion (13) of a treatment apparatus (200), including
at least one hinge axis (3), whereby the cover member (1) may rotatably be fastened to the treatment apparatus (200);
wherein the cover means (100) comprises at least one connection means (5), whereby the hinge axis (3) may be latched with the cover member (1) and
wherein the connection means (5) comprises at least one bush means (9) for receiving or mounting at least one portion of the hinge axis (3), wherein the connection means (5) is provided on the side of the cover member (1) and the hinge axis (3) is provided on the side of the treatment apparatus (200),
**characterized in that**,
in a closed condition of the cover member (1), the bush means (9) encompasses the hinge axis (3) from the side of the treatment apparatus (200).

2. The cover means (100) according to claim 1, wherein the cover member (1) may be, or is, connected to the treatment apparatus (200) in a nondestructive releasable manner.

3. The cover means (100) according to any one of the preceding claims, wherein the hinge axis (3) is configured for being latched into an opening (11).

4. The cover means (100) according to any one of the preceding claims, wherein at least the cover member (1) is transparent in at least one portion thereof.

5. Treatment apparatus (200), **characterized by** a cover means (100) according to any one of claims 1 to 4.

6. The treatment apparatus (200) according to claim 5, comprising at least one stator (7) which is adapted and provided to accommodate at least one part of a hinge axis (3) of the cover means (100).

7. The treatment apparatus (200) according to claim 5 or 6, which is configured as a blood treatment apparatus.

8. The treatment apparatus (200) according to claim 7, which is configured as a dialyzing apparatus.

9. The treatment apparatus (200) according to claim 8, which is configured as a hemodialyzing apparatus.

10. The treatment apparatus (200) according to any one of claims 7 to 9, wherein the functional portion (13) is part of an extracorporeal blood circulation.

11. The treatment apparatus (200) according to claim 10, wherein the functional portion (13) of the treatment apparatus (100) is a reception portion for a tubing segment of a blood pump.

12. The treatment apparatus (200) according to claim 10, wherein the functional portion (13) is a sensor.

13. A cover member (1) for a cover means (100) according to any one of claims 1 to 4.

14. The cover member (1) according to claim 13, which comprises at least one connection means (5) for latching it with a hinge axis (3).

15. A method of replacing a cover member (1) of a treatment apparatus (200), wherein the treatment apparatus (200) comprises at least one cover means (100) according to any one of claims 1 to 4, **characterized by** the step of:
- fastening a replacement cover member (1) by latching at least one hinge axis (3) with at least one connection means (5) for rotatably fastening the cover member (1) to the treatment apparatus (200).

## Revendications

1. Dispositif de protection (100) avec au moins un élément de protection (1) pour recouvrir au moins une partie fonctionnelle (13) d'un équipement de traitement (200), comprenant
au moins un axe de charnière (3) au moyen duquel l'élément de protection (1) est fixable de manière rotative à l'équipement de traitement (200), où le dispositif de protection (100) présente au moins un élément de connection (5) au moyen duquel l'axe de charnière (3) peut être encliqueté dans l'élément de protection (1), et où
l'élément de connection (5) présente au moins un dispositif de douille (9) pour recevoir au moins une section de l'axe de charnière (3) ou pour son entreposage, où l'élément de connection (5) est prévu du côté de l'élément de protection (1) et l'axe de charnière (3) est prévu du côté de l'équipement de traitement (200),
**caractérisé en ce que**,
pour une configuration fermée de l'élément de protection (1), le dispositif de douille (9) est engagé autour de l'axe de charnière (3) du côté de l'équipement de traitement (200).

2. Le dispositif de protection (100) selon la revendication 1, où l'élément de protection (1) peut être relié ou est relié avec l'équipement de traitement (200) de manière détachable et non destructive.

3. Le dispositif de protection (100) selon l'une quelconque des revendications précédentes, où l'axe de charnière (3) est formé de façon à pouvoir être encliquité dans une ouverture (11).

4. Le dispositif de protection (100) selon l'une quelconque des revendications précédentes, où au moins l'élément de protection (1) est transparent dans au moins une de ses sections.

5. Equipement de traitement (200), **caractérisé par** un dispositif de protection (100) selon l'une quelconque des revendications 1 à 4.

6. L'équipement de traitement (200) selon la revendication 5, présentant au moins un stator (7) prévu pour et formé de façon à recevoir au moins une portion d'un axe de charnière (3) du dispositif de protection (100).

7. L'équipement de traitement (200) selon la revendication 5 ou 6 formé comme équipement de traitement pour le sang.

8. L'équipement de traitement (200) selon la revendication 7 formé comme équipement de dialyse.

9. L'équipement de traitement (200) selon la revendication 8 formé comme équipement d'hemodialyse.

10. L'équipement de traitement (200) selon l'une des revendications 7 à 9, où la partie fonctionnelle (13) fait part d'un circuit sanguin extracorporel.

11. L'équipement de traitement (200) selon la revendication 10, où la partie fonctionnelle (13) de l'équipement de traitement (100) est une portion de réception pour un segment de flexible d'une pompe à sang.

12. L'équipement de traitement (200) selon la revendication 10, où la partie fonctionnelle (13) est un capteur.

13. Un élément de protection (1) pour un dispositif de protection (100) selon l'une quelconque des revendications 1 à 4.

14. L'élément de protection (1) selon la revendication 13, qui présente au moins un élément de connection (5) pour son encliquetage avec un axe de charnière (3).

15. Une méthode de remplacement d'un élément de protection (1) d'un équipement de traitement (200), où l'équipement de traitement (200) présente au moins un dispositif de protection (100) selon l'une quelconque des revendications 1 à 4, **caractérisée par** l'étape de:
- fixer un élément de protection (1) de remplacement en encliquetant au moins un axe de charnière (3) avec au moins un élément de connection (5) pour fixer l'élément de protection (1) de manière rotative à l'équipement de traitement (200).
